# FASCICULE DE BREVET EUROPEEN

(11) **EP 4 336 990 B1**
(45) Date de publication et mention de la délivrance du brevet: **29.04.2026**
(21) Numéro de dépôt: 22315236.4
(22) Date de dépôt: 11.10.2022
(51) Int. Cl.: H10N 30/30, H10N 30/03, H02N 2/18, A61N 1/378

(54) **ENSEMBLE PENDULAIRE À MASSE INERTIELLE MONOLITHIQUE MONTÉ SUR UNE LAME PIÉZOÉLECTRIQUE, NOTAMMENT POUR UN RÉCUPÉRATEUR D'ÉNERGIE DE CAPSULE CARDIAQUE AUTONOME LEADLESS**
PENDELANORDNUNG MIT AUF EINEM PIEZOELEKTRISCHEN BALKEN MONTIERTEM MONOLITHISCHEM TRÄGHEITSGEWICHT, INSBESONDERE FÜR EINE ENERGIEGEWINNUNGSVORRICHTUNG EINER DRAHTLOSEN AUTONOMEN KARDIOKAPSEL
PENDULAR ASSEMBLY WITH MONOLITHIC INERTIAL MASS MOUNTED ON A PIEZOELECTRIC BEAM, IN PARTICULAR FOR AN ENERGY HARVESTER OF A LEADLESS AUTONOMOUS CARDIAC CAPSULE

(43) Date de publication de la demande: 13.03.2024
(73) Titulaire: CAIRDAC, 92160 Antony (FR)
(72) Inventeur: Regnier, Willy, 91160 LONGJUMEAU (FR); Nguyen-Dinh, An, 37520 La Riche (FR)
(74) Mandataire: Dupuis-Latour, Dominique

(56) Documents cités:
- WO-A1-2005/067073
- JP-A- 2011 066 970
- US-A- 3 456 134
- US-A1- 2019 381 325

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" (*Piezoelectric Energy Harvester*)*,* qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en œuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless",* pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs autonomes, quelle que soit leur destination fonctionnelle, cardiaque ou autre, médicale ou non.

Dans le cas d'une application cardiaque, la capsule leadless surveille en continu le rythme du patient et délivre si nécessaire au cœur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique ou "PZT" (titano-zirconate de plomb) et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entrainée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique en flexion de manière à engendrer au sein du matériau qui le constitue des charges électriques qui sont récupérées en surface du composant pour être utilisées par le système d'autoalimentation de la capsule leadless. Le PZT se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continu stabilisés permettant d'assurer l'alimentation des divers circuits électroniques et capteurs de la capsule, ainsi que la recharge de l'organe de stockage d'énergie.

La structure mécanique d'un tel récupérateur d'énergie de type PEH est notamment décrite en détail dans le WO 2018/122244 A1 (Sorin CRM/Regnier).

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Dans les structures de PEH proposées jusqu'à présent, par exemple par le WO 2018/122244 A1 précité, la masse inertielle est constituée de deux demi-masses identiques, agencées symétriquement de part et d'autre de la lame PZT. Ces deux demi-masses forment ensemble un tronc de cône et sont fixées à l'extrémité libre de la lame de chaque côté de celle-ci par collage.

La conicité de la surface extérieure de la masse inertielle permet d'optimiser l'espace disponible avant d'entrer en contact avec l'intérieur du tube contenant le PEH. Cette géométrie n'est toutefois pas exhaustive et peut être adaptée à son environnement pour optimiser le rapport masse/encombrement.

Le matériau utilisé pour la masse inertielle est un métal, généralement un tungstène moulé, qui présente une masse volumique élevée pour un coût de revient maîtrisé, et les dimensions de la masse sismique sont ajustées en fonction du poids final nécessaire au mode vibratoire souhaité, compte tenu de la géométrie et de l'élasticité de la lame PZT.

D'autres structures de PEH sont illustrées notamment dans les US 3 456 134 A (Ko), JP 2011 066970 A (Sumida Corp.) ou WO 2005/067073 A1 (Pirelli Pneumatici SpA).

Le problème de l'invention trouve sa source dans les difficultés rencontrées du fait du mode d'assemblage de la masse inertielle à la lame PZT, exécuté par collage du métal de chacune des demi-masses sur les faces de la lame PZT en céramique.

En premier lieu, la présence d'une matière chimique de liaison à l'interface métal/céramique a une incidence sur la durée de vie du PEH. Même avec une maîtrise parfaite des colles et de leur mise en œuvre, il n'a pas jusqu'à présent été possible de garantir une durée de vie supérieure à dix ans sans risque de défaillance.

Ce chiffre de dix ans (correspondant à environ 300 millions de cycles cardiaques) est celui habituellement retenu pour les stimulateurs cardiaques conventionnels, dont il est de toute façon nécessaire de remplacer le générateur à cette échéance compte tenu de l'épuisement de la pile intégrée d'alimentation ; en revanche, dans le cas d'un stimulateur leadless, difficile à explanter pour le remplacer par un appareil neuf, il serait nécessaire de pouvoir garantir une longévité bien supérieure, typiquement de 20 ans de fonctionnement continu sans défaillance.

Les colles employées jusqu'à présent ne permettent toutefois pas de garantir une telle performance, même pour celles qui ne se dégradent que très peu au cours du temps.

Un deuxième problème réside dans la difficulté qu'il y a à bien maîtriser le processus de collage au moment de la fabrication du PEH. Ce processus est en soi très délicat à mettre en œuvre du fait des dimensions très réduites des pièces, de la nécessité d'opérer sous atmosphère contrôlée, et d'éviter toute pollution chimique par des contaminants susceptibles de modifier les propriétés de tenue au vieillissement du collage effectué.

Un troisième problème réside, lors de ce processus de collage, dans la difficulté particulière qu'il y a à maîtriser parfaitement la quantité de colle utilisée : une quantité insuffisante de colle diminue bien évidemment la solidité du collage final obtenu, mais à l'inverse un excès de colle se traduit par un épanchement de la colle au-delà de l'interface métal/céramique, avec un risque d'altérer la flexibilité de la lame PZT (qui perd sa flexibilité à l'endroit où la colle a débordé) avec augmentation de la fréquence de vibration propre de l'ensemble pendulaire, et par voie de conséquence un dérèglement du système induisant une moindre récupération d'énergie par le PEH, toutes choses égales par ailleurs.

Le but de l'invention est de proposer une nouvelle structure de module PEH, et un nouveau procédé d'assemblage d'une telle structure, qui pallient les difficultés et limitations que l'on vient d'exposer, en permettant notamment :
· de garantir une longévité du PEH pouvant atteindre 20 ans ;
· d'offrir une technique de montage simplifiée, économique et non opérateur-dépendante ; et
· d'obtenir un PEH avec des caractéristiques vibratoires parfaitement maîtrisées et, de fait, un rendement optimisé de la fonction de récupération d'énergie.

### RÉSUMÉ DE L'INVENTION

Pour résoudre ces problèmes et atteindre les buts exposés ci-dessus, l'invention propose un ensemble pendulaire destiné à un module PEH tel que défini dans les revendications 1 à 9 annexées.

L'invention a également pour objet un procédé d'assemblage d'un tel ensemble pendulaire tel que défini dans les revendications 10 à 12 annexées, ainsi qu'un PEH comprenant un ensemble pendulaire tel que ci-dessus selon la revendication 13, et un dispositif autonome incorporant dans un corps de dispositif un tel PEH selon la revendication 14.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du cœur d'un patient.
La Figure 2 illustre une capsule leadless implantée au fond du ventricule droit d'un patient.
La Figure 3 montre isolément un ensemble pendulaire de type connu, avec un PZT en forme de lame allongée encastrée à une extrémité et supportant une masse inertielle à son extrémité opposée.
La Figure 4 présente sous forme schématique les principaux blocs fonctionnels constitutifs d'une capsule leadless.
La Figure 5 est une vue en coupe transversale, par un plan axial, du module PEH selon l'invention.
La Figure 6 est une vue éclatée en perspective montrant les différents éléments constitutifs du module PEH de la Figure 5.
La Figure 7 est une vue en coupe, par un plan axial, d'une masse inertielle d'un ensemble pendulaire non couvert en tant que tel par les revendications de la présente invention.
La Figure 8 est une vue en coupe, par un plan axial, d'une masse inertielle d'un ensemble pendulaire selon un premier mode de réalisation de l'invention.
La Figure 9 est une vue en coupe, par un plan axial, d'une masse inertielle d'un ensemble pendulaire selon un deuxième mode de réalisation de l'invention.
La Figure 10 est une vue en coupe, par un plan axial, d'une masse inertielle d'un ensemble pendulaire selon un troisième mode de réalisation de l'invention.
La Figure 11 est une vue en coupe, par un plan radial selon XI-XI de la Figure 10, de la masse inertielle de l'ensemble pendulaire de la Figure 10.
La Figure 12 est une vue perspective montrant, isolément, la configuration de l'extrémité de la lame PZT de l'ensemble pendulaire de la Figure 10.
La Figure 13 est une vue perspective, partiellement en coupe, de l'ensemble pendulaire de la Figure 10.
La Figure 14 est une vue perspective montrant sous forme assemblée finale l'ensemble pendulaire de la Figure 10.
Les Figures 15 et 16 illustrent deux étapes d'assemblage d'une capsule leadless avec un module PEH comprenant un ensemble pendulaire selon l'invention.
La Figure 17 illustre la capsule leadless implantable finale obtenue en fin de processus.
La Figure 18 est un organigramme explicitant les différentes étapes du processus d'assemblage d'une capsule leadless implantable comprenant un module PEH avec un ensemble pendulaire selon l'invention.

### DESCRIPTION DÉTAILLÉE DE MODES DE RÉALISATION

### PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

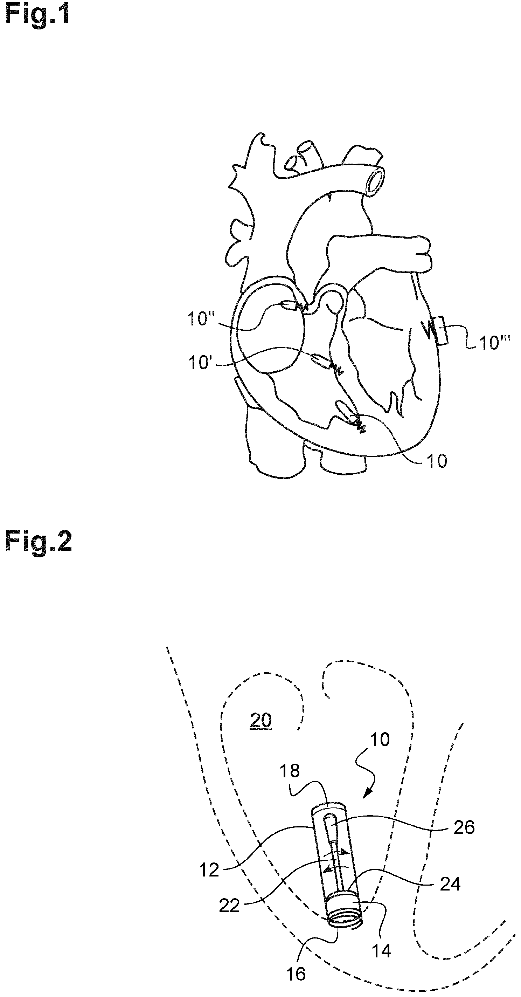

Sur la Figure 1, on a représenté diverses possibilités de sites d'implantation d'un dispositif de type leadless, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10‴.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en œuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en œuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en œuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

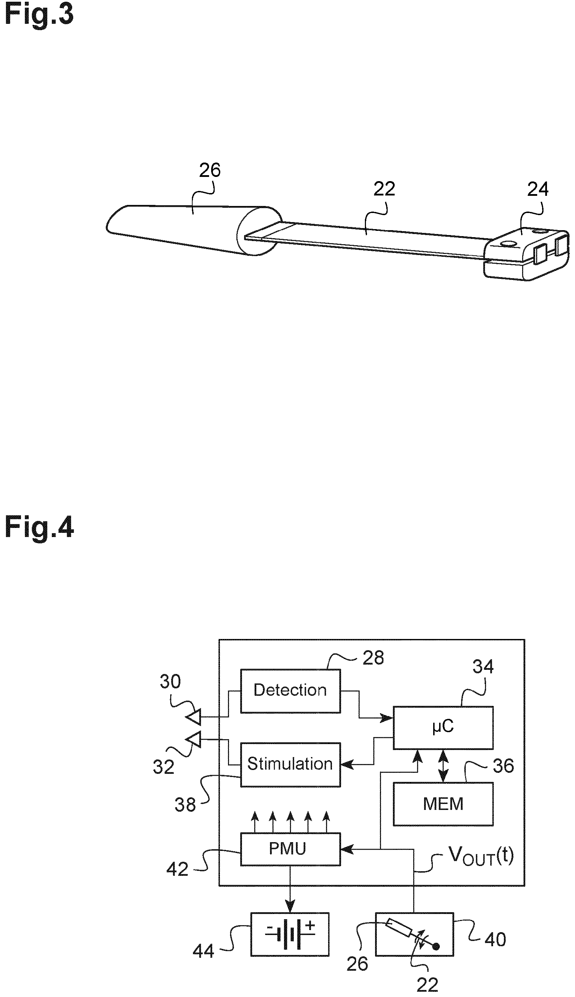

L'ensemble pendulaire, illustré isolément Figure 3, est constitué d'une lame piézoélectrique 22 assujettie à une pièce d'encastrement 24 à l'une de ses extrémités (ci-après "extrémité proximale" de la lame) et dont l'extrémité opposée, libre (ci-après "extrémité distale" de la lame) est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames PZT des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et · électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continu stabilisés servant à l'alimentation des divers circuits électroniques et à la recharge de la batterie intégrée 44.

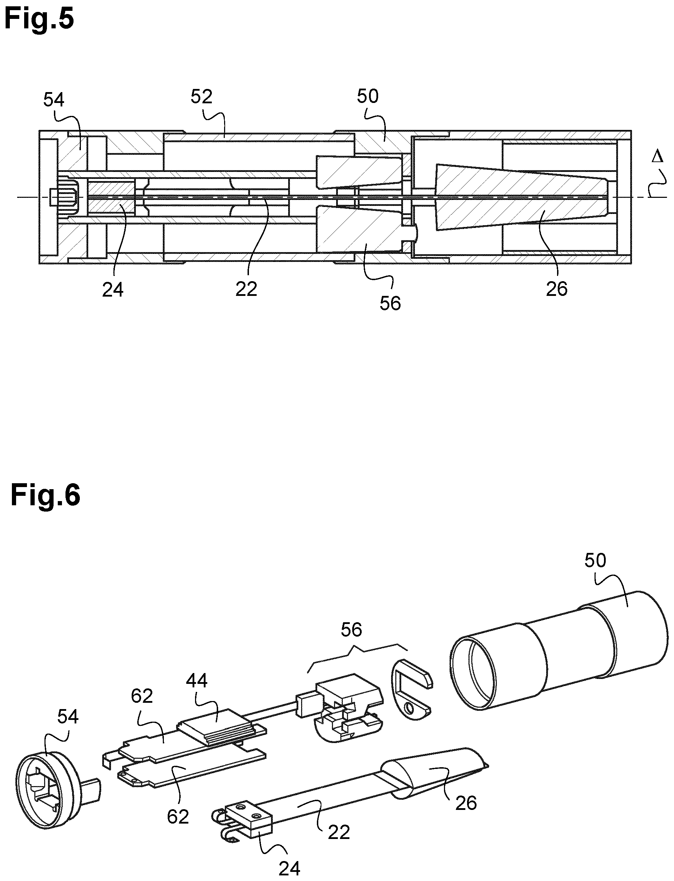

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. Sur les Figures 5 et 6 on a représenté les principaux éléments constitutifs d'un module PEH selon l'invention.

Ces différents éléments sont logés à l'intérieur d'un tube enveloppe 50, qui est généralement un tube métallique (pour permettre des opérations de soudage qui seront décrites plus bas), de préférence en titane en raison de l'excellente biocompatibilité de ce métal.

Un tube enveloppe particulièrement adapté à la réalisation d'une capsule leadless est décrit notamment dans le EP 3 730 185 A1 (Cairdac), correspondant au US 2020/338241 A1 (Regnier et al.), qui illustre notamment un tube composite métal/céramique comprenant une partie centrale (52 sur la Figure 5) en matériau céramique transparent aux radiofréquences, de manière à permettre une communication sans fil entre des circuits électroniques situés à l'intérieur du tube et l'environnement extérieur, le reste du tube étant en matériau métallique tel que le titane et le tout formant un ensemble·monobloc tubulaire.

Le tube enveloppe 50 loge l'ensemble pendulaire constitué de la lame 22 maintenue côté proximal par la pièce d'encastrement 24 et portant côté distal la masse inertielle 26. L'ensemble pendulaire est agencé au centre du tube enveloppe 50 et aligné sur l'axe Δ du tube.

Dans la suite, on entendra par "direction axiale" la direction de plus grande longueur de la lame et par "direction transversale" la direction de déformation de la lame, direction qui est située dans un plan radial et qui est perpendiculaire à la direction axiale Δ ; la direction perpendiculaire aux directions axiale et transversale sera dénommée "direction latérale".

La pièce d'encastrement 24 est maintenue dans le tube par une monture 54 assujettie au tube, notamment une monture en un matériau métallique tel que le titane, susceptible d'être soudé au tube en périphérie de manière à assujettir au tube 50 la monture 54, et donc l'encastrement 24 et la lame 22.

Le EP 3 892 325 A1 (Cairdac), correspondant au US 2021/316148 A1 (Regnier et al.), décrit en détail un exemple de pièce d'encastrement et de monture, et on pourra se référer à ce document pour de plus amples détails.

Le tube 50 loge également une ou plusieurs cartes de circuit imprimé (PCB) 62, dans l'exemple illustré deux PCB 62 dont l'un porte la batterie 44. Ces deux PCB sont reliés l'un à l'autre par une nappe de conducteurs flexibles et supportés à chacune de leurs extrémités respectivement côté distal par un insert 56 et côté proximal par la monture 54.

La configuration de ces PCB de part et d'autre de la lame 22, et la manière dont ils sont réunis par une nappe flexible et supportés entre un élément proximal et un élément distal sont décrits notamment dans le US 2019/381325 A1 précité, auquel on pourra se référer pour de plus amples détails.

L'insert 56 est par exemple un insert de symétrisation tel que celui décrit dans le EP 4 276 923 A1 au nom du demandeur (non publié à la date de dépôt de la présente demande). Cet insert de symétrisation permet de préserver l'amplitude maximale d'oscillation de la lame en évitant qu'elle ne soit réduite par un positionnement sous-optimal de l'ensemble pendulaire dans le corps du module, notamment du fait d'un positionnement imparfait (décentrage, désalignement) de la masse inertielle 26.

L'invention concerne plus précisément la manière dont est réalisée et assemblée la masse inertielle 26 de l'ensemble pendulaire de la capsule leadless que l'on vient de décrire.

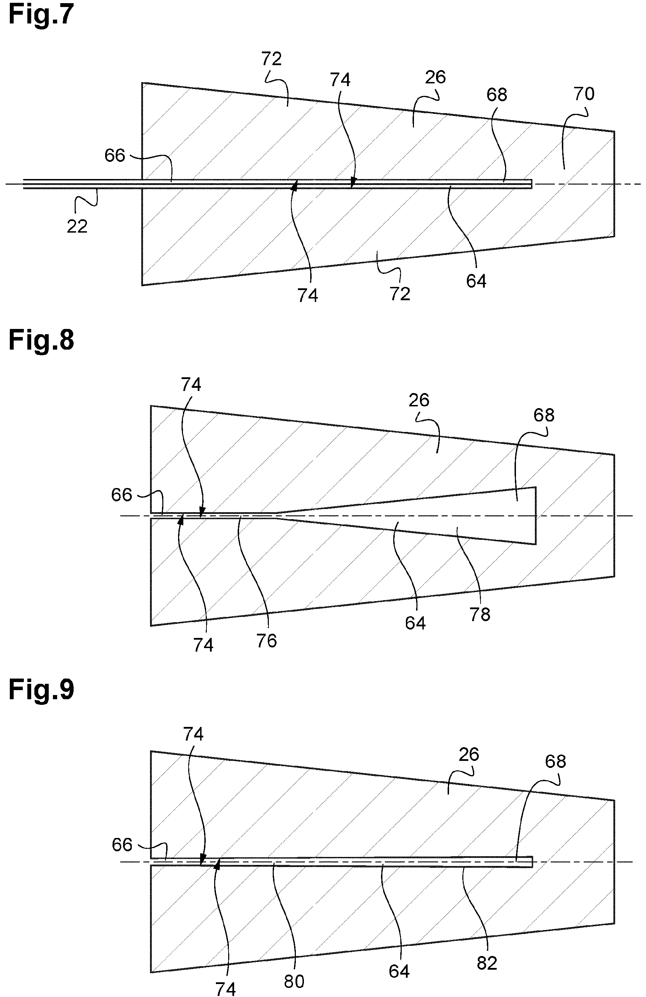

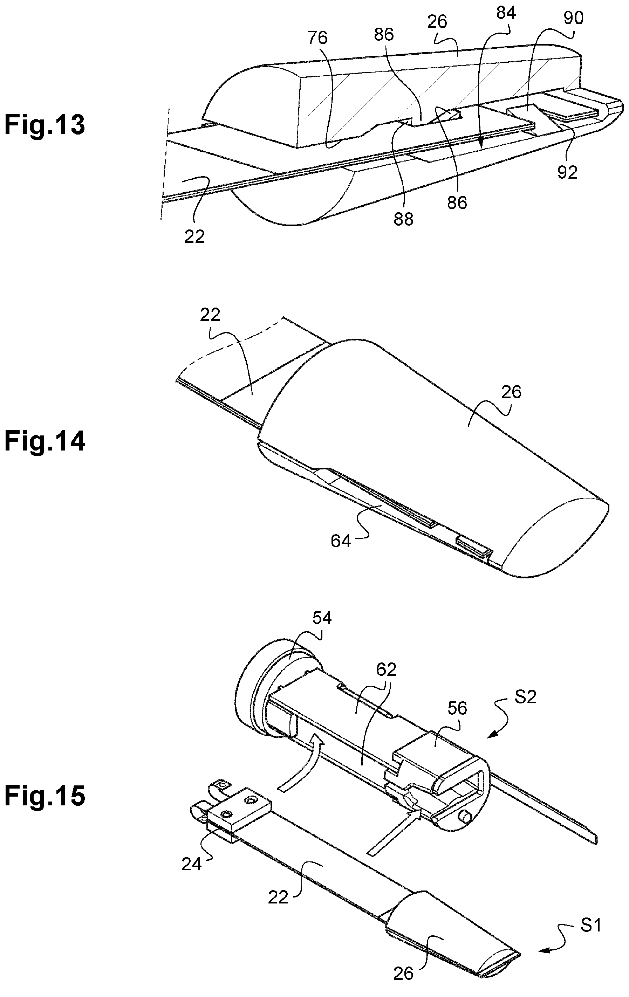

Les Figures 8 à 14 illustrent divers modes de réalisation selon l'invention, et les Figures 15 à 18 illustrent le procédé de réalisation d'une capsule leadless munie d'un tel ensemble pendulaire.

La Figure 7 illustre un ensemble pendulaire, non couvert en tant que tel par les revendications de la présente invention, dans une vue en coupe par un plan axial.

La masse inertielle 26 est formée d'une pièce unique, monobloc, usinée dans la masse, de forme approximativement tronconique. Dans cette pièce a été formée une cavité 64 en forme de fente axiale, typiquement par une technique connue d'usinage par enlèvement de matière au moyen d'un disque qui retire la matière au centre de la pièce de façon transversale, ou encore par une technique d'électroérosion à fil.

En variante, il est possible de former la cavité 64 par des techniques additives de dépôt de matière telles que stéréolithographie (SLA), frittage sélectif par laser (SLS) ou dépôt de fil fondu (FDM) par exemple.

La matière constituant la masse inertielle 26 est avantageusement le tungstène (ou encore le platine, l'osmium, l'or, l'iridium ou toute autre matière à forte densité volumique), ce qui permet d'obtenir une masse relativement élevée sous un volume réduit.

La fente formant la cavité axiale 64 s'étend d'une extrémité proximale 66 débouchante jusqu'à une extrémité distale 68 borgne, de manière à laisser subsister à cette extrémité 68 une partie intacte 70, non usinée, au fond de la cavité. Une fois usinée, la pièce se présente alors avec une région d'âme correspondant à la partie intacte 70 à partir de laquelle s'étendent deux branches 72, le tout formant une pièce monobloc. La fente formant la cavité axiale 64 définit deux faces opposées intérieures en vis-à-vis 74, 74 formant des surfaces d'appui permettant de prendre en sandwich l'extrémité distale de la lame PZT 22. Dans ce mode de réalisation, les deux surfaces 74, 74 de la cavité 64 sont parallèles sur toute la longueur de la fente, de l'extrémité proximale 66 jusqu'à l'extrémité distale 68.

Pour permettre un pincement de la lame, la hauteur de la fente entre les surfaces 74 en vis-à-vis est légèrement inférieure, à un jeu fonctionnel près, à l'épaisseur de la lame PZT 22 qui sera introduite après l'usinage de la fente formant la cavité axiale 64, de manière à contraindre l'assemblage par un effort permanent sur la partie (ou la totalité) des surfaces 74 en contact avec les surfaces externes correspondantes de la lame PZT 22.

On peut ainsi obtenir un assemblage permanent de l'ensemble sans ajout de matière, notamment sans ajout de colle et sans qu'il soit nécessaire de prévoir des pièces rapportées pour assujettir la masse inertielle 26 à la lame PZT 22.

Cette opération de montage de la lame PZT 22 dans la cavité 64 de la masse inertielle 26 peut être réalisée par diverses techniques telles que :
· déformation en température de la lame PZT 22 et/ou de la masse inertielle 26 avant insertion de la lame PZT 22 dans la cavité 64, puis retour de l'ensemble à température ambiante après insertion ;
· déformation élastique de la masse inertielle 26 par écartement forcé des deux branches 72 pour agrandir la fente 64 (en écartant les surfaces internes 74 en vis-à-vis) avant l'insertion de la lame PZT 22, puis relâchement après insertion de cette dernière ;
· collage, sertissage, et/ou soudure d'une pièce rapportée de liaison permettant d'immobiliser entre elles la lame PZT 22 et la masse inertielle 26 (les deux techniques précédentes étant toutefois préférées).

La Figure 8 est homologue de la Figure 7, mais dans un premier mode de réalisation, spécifique à l'invention.

Dans ce mode de réalisation, afin de réduire les surfaces en contact avec la lame, la fente forme des cavités successives avec des géométries différentes. Dans l'exemple illustré, la cavité 64 comprend une première partie 76 avec deux surfaces en vis-à-vis 74, 74 planes et parallèles (comme dans le cas de la Figure 7), mais la longueur en direction axiale à partir de l'extrémité proximale 66 de cette première partie est plus réduite, et la cavité s'élargit ensuite à l'endroit d'une seconde partie 78, jusqu'à l'extrémité distale 68.

Cette configuration permet de disposer de deux zones fonctionnellement différentes, avec une zone de serrage correspondant à la partie la plus proximale 76, et une zone sans serrage 78 correspondant au reste de la longueur de la cavité. L'effort de pincement de la lame PZT 22 s'exercera seulement dans la région 76 entre les deux surfaces d'appui parallèles 74, 74. Cet effort étant plus important (surface en contact plus réduite), il conviendra de contrôler la pression exercée sur I lame PZT de manière à ne pas endommager celle-ci, en limitant l'effort à une valeur typiquement comprise entre 0,5 et 2 N/mm².

La géométrie multicavité de ce premier mode de réalisation pourra être réalisée par des techniques connues telles que l'électroérosion à fil ; en ce qui concerne la solidarisation de la lame PZT à la masse inertielle 26 on pourra utiliser les diverses techniques précédemment exposées à propos de la Figure 7.

La Figure 9 est homologue des Figures 7 et 8, pour un deuxième mode de réalisation de l'invention.

Dans ce mode de réalisation, les surfaces en vis-à-vis 74, 74 sont des surfaces planes, mais qui ne sont pas parallèles : la largeur la fente est progressivement croissante depuis l'extrémité proximale 66 jusqu'à l'extrémité distale 68. À l'extrémité proximale 66, l'écartement en direction radiale entre les surfaces 74, 74 est inférieur à l'épaisseur de la lame PZT à un jeu négatif près, tandis qu'à l'extrémité distale 68 cet écartement n'est pas inférieur à l'épaisseur de la lame.

Une fois la lame PZT introduite dans la cavité 64 (par l'une des techniques exposées précédemment), les deux surfaces en vis-à-vis 74, 74 épousent la forme de la lame et se retrouvent dans une configuration parallèle, en imposant un serrage progressif de la masse inertielle 26 sur la lame, l'effort de serrage étant maximal dans la région 80 à l'extrémité proximale 66 et nul dans la région 82 à l'extrémité distale 68.

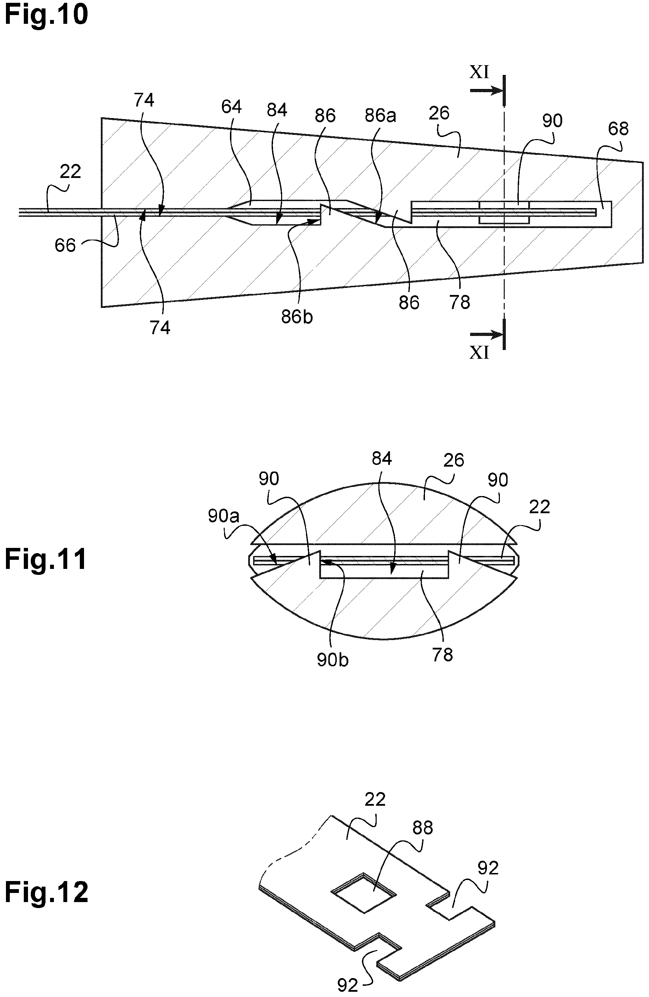

Les Figures 10 à 14 illustrent un troisième mode de réalisation de l'invention.

Dans ce mode de réalisation la cavité axiale 64 comporte, comme dans le mode de réalisation illustré Figure 8, deux parties distinctes, avec côté proximal une partie comportant les deux faces 74, 74 en vis-à-vis formant surfaces d'appui, destinées à prendre en sandwich et à immobiliser la lame PZT 22. Dans le reste de la cavité, une partie élargie 78 forme jusqu'à l'extrémité distale 68 une zone sans serrage, recevant la lame PZT mais n'exerçant pas sur celle-ci de pression de serrage.

Dans la partie élargie 78, les faces en vis-à-vis 84 de la cavité 64 sont pourvues de géométries aptes à immobiliser par effet de crantage, axialement et radialement, la masse inertielle 26 par rapport à la lame PZT 22. Ces géométries sont par exemple formées de crans 86, 90 avec, dans l'exemple illustré et de façon non limitative, deux crans d'immobilisation axiale 86 orientés en sens opposés, et deux crans d'immobilisation radiale 90, également orientés en sens opposés.

Les crans d'immobilisation axiale 86 comprennent chacun une face inclinée 86a en forme de rampe orientée en direction axiale, vers l'avant pour l'un des crans 86 et vers l'arrière pour l'autre cran 86, ces rampes se terminant par une face abrupte 86b permettant de réaliser l'effet de crantage recherché. Ces crans d'immobilisation axiale 86 coopèrent avec une découpe (ouverture ou encoche) conjuguée 88 réalisée dans la lame PZT à l'endroit où les crans vont venir se placer dans la position définitive d'immobilisation.

De façon comparable, les crans 90 d'immobilisation radiale comprennent chacun une face inclinée 90a en forme de rampe orientée en direction radiale, dans un sens pour l'un des crans 90 et dans le sens opposé pour l'autre cran 90, ces rampes se terminant par une face abrupte 90b permettant d'obtenir l'effet de crantage avec la lame PZT à l'endroit de découpe conjuguée 92 formée dans cette dernière.

Ces géométries de crantage permettent en particulier la mise en position progressive des composants (masse inertielle 26 et lame PZT 22) au moment de leur montage, avec un effet de blocage final bidimensionnel axial et bidimensionnel radial ; la lame PZT 22, qui n'est pas comprimée dans la partie 78 élargie sans serrage, est libre de se déplacer jusqu'à son encliquetage final.

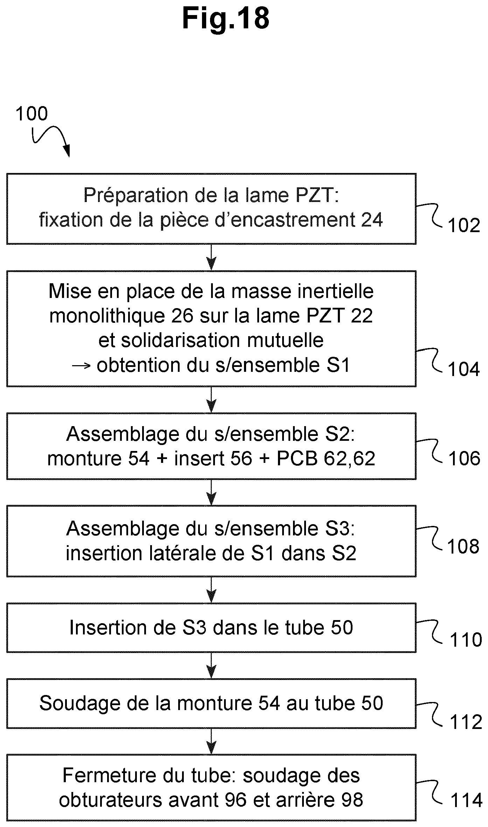

En référence aux Figures 15 à 17 et à l'organigramme de la Figure 18 présentant les différentes étapes du processus, on va maintenant exposer la manière dont sont fabriqués et assemblés le module PEH comprenant l'ensemble pendulaire muni de la masse sismique que l'on vient de décrire, ainsi que la capsule leadless complète intégrant un tel module. La première étape (bloc 102 de l'organigramme 100 de la Figure 18) consiste à préparer la lame PZT et à fixer la pièce d'encastrement 24 à son extrémité proximale.

L'étape suivante (bloc 104) consiste à mettre en place sur la lame PZT 22 la masse inertielle monolithique 26 préalablement usinée comme décrit plus haut, la lame PZT 22 étant prise en sandwich à l'intérieur de la cavité axiale 64, et ce jusqu'à solidarisation mutuelle de la masse inertielle 26 et de la lame PZT 22 selon l'une des diverses techniques décrites plus haut. On obtient ainsi un premier sous-ensemble S1 (Figure 15) constitué de la lame PZT 22, de la pièce d'encastrement 24 à son extrémité proximale, et de la masse inertielle 26 à son extrémité distale.

L'étape suivante (bloc 106) consiste à assembler un sous-ensemble S2 (Figure 15) regroupant la monture 54, l'insert 56, et les deux PCB 62 montés entre ces deux éléments 54 et 56.

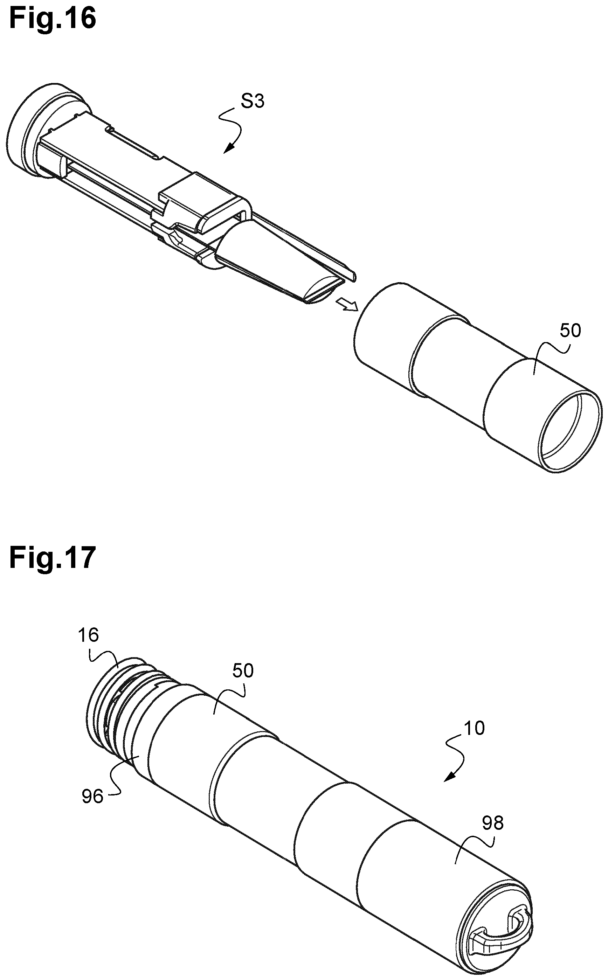

L'étape suivante (bloc 108) consiste à réunir les sous-ensembles S1 et S2 en un sous-ensemble S3 (Figure 16) par introduction du sous-ensemble S1 dans l'espace latéral ménagé entre l'insert 56, la monture 54 et les deux PCB 62 du sous-ensemble S2.

L'étape suivante (bloc 110) consiste à introduire ce sous-ensemble S3 dans le tube enveloppe 50 par translation axiale (Figure 16). La monture 54 est alors soudée au tube 50 (étape 112), par exemple au moyen de tirs laser périphériques.

L'étape finale (bloc 114) consiste à fermer à ses deux extrémités le tube enveloppe 50 contenant l'ensemble pendulaire, par ajout d'un obturateur avant 96 portant la vis d'ancrage 16 de la capsule leadless, et d'un obturateur arrière 98. Ces obturateurs 96 et 98 sont fixés au tube 50 par des soudages laser périphériques. La capsule leadless se présente alors dans son état assemblé final, tel qu'illustré sur la Figure 17.

## Revendications

1. Un ensemble pendulaire destiné à un module de récupération d'énergie, PEH, l'ensemble pendulaire comprenant :
- une lame de transducteur piézoélectrique ou titano-zirconate de plomb, PZT, (22) élastiquement déformable en flexion et s'étendant en direction axiale entre une extrémité proximale encastrée et une extrémité distale libre ; et
- une masse inertielle (26), montée à l'extrémité distale libre de la lame PZT (22) et mobile en direction transversale,
l'ensemble pendulaire étant apte à convertir une énergie mécanique produite par des oscillations de l'ensemble pendulaire sous l'effet de sollicitations externes subies par le module en un signal électrique oscillant recueilli par des électrodes de surface de la lame PZT (22), dans lequel la masse inertielle (26) est une pièce monolithique comportant une cavité en forme de fente axiale (64), avec deux surfaces (74) longitudinales opposées s'étendant le long d'un axe central de la masse inertielle (26), la fente axiale (64) débouchant côté proximal de la masse inertielle (26) et logeant l'extrémité distale libre de la lame PZT (22), **caractérisé en ce que** ladite cavité en forme de fente axiale (64) comprend :
- côté proximal (66), une zone de serrage (76 ; 80) où la lame PZT (22) est assujettie entre les deux surfaces longitudinales opposées (74) de la fente axiale ; et
- côté distal (68), une zone sans serrage (78 ; 82).

2. L'ensemble pendulaire de la revendication 1, dans lequel, sur la longueur de la zone de serrage (76), les deux surfaces longitudinales opposées (74) comprennent des surfaces symétriques planes et parallèles séparées par un écartement radial constant.

3. L'ensemble pendulaire de la revendication 2, dans lequel les deux surfaces longitudinales opposées (74) comprennent des surfaces s'élargissant à l'endroit de la longueur de la zone sans serrage (78).

4. L'ensemble pendulaire de la revendication 1, dans lequel les deux surfaces longitudinales opposées (74) sont des surfaces symétriques séparées radialement par un écartement radial croissant, dans la direction proximale vers distale, sur au moins une partie de la longueur de la fente axiale (64) en direction longitudinale, de manière à produire un serrage progressif de la lame PZT (22), avec un effort de serrage maximal dans la zone de serrage (80) et nul dans la zone sans serrage (82).

5. L'ensemble pendulaire de la revendication 1, dans lequel, en direction radiale, la fente axiale (64) débouche également sur au moins l'un des côtés de la masse inertielle (26).

6. L'ensemble pendulaire de la revendication 1, dans lequel au moins l'une des deux surfaces longitudinales opposées (74) comprend au moins un cran anti-retour (86, 90) muni d'une butée axiale (86b) et/ou radiale (90b) apte à bloquer en place la lame PZT (22) dans la fente axiale (64).

7. L'ensemble pendulaire de la revendication 6, dans lequel la lame PZT (22) comprend, dans une région comprise entre les surfaces longitudinales opposées (74) de la fente axiale (64), au moins une découpe (88, 92) apte à coopérer avec une butée axiale (86b) et/ou radiale (90b) homologue d'un cran anti-retour (86, 90) de la masse inertielle (26).

8. L'ensemble pendulaire de la revendication 1, dans lequel, dans la zone de serrage (76), la valeur minimale de l'écartement radial entre les surfaces longitudinales opposées (74) est égale à l'épaisseur de la lame PZT (22) à un jeu négatif près, de manière à exercer sur la lame PZT (22) un effort de pincement entre les surfaces opposées.

9. L'ensemble pendulaire de la revendication 8, dans lequel l'effort de pincement de la lame PZT (22) par les surfaces longitudinales opposées (74) est compris entre 0,5 et 2 N/mm².

10. Un procédé d'assemblage d'un ensemble pendulaire destiné à un module de récupération d'énergie, PEH, l'ensemble pendulaire comprenant une lame de transducteur piézoélectrique ou titano-zirconate de plomb, PZT, (22) élastiquement déformable en flexion et une masse inertielle (26) montée à l'extrémité distale libre de la lame PZT (22) et mobile en direction transversale, comportant les étapes suivantes :
a) obtention d'une masse inertielle (26) par formation d'une fente axiale (64) dans la masse d'une pièce monolithique, la fente axiale (64) s'étendant le long d'un axe central de la masse inertielle (26) à partir d'une extrémité proximale en formant deux surfaces longitudinales opposées (74),
la fente axiale (64) comprenant des cavités successives avec des géométries différentes, comprenant : côté proximal (66), une zone de serrage (76 ; 80) où la lame PZT (22) est assujettie entre les deux surfaces longitudinales opposées (74) de la fente axiale ; et côté distal (68), une zone sans serrage (78 ; 82) ;
b) insertion dans la fente axiale (64) de l'extrémité distale libre d'une lame PZT (22) ; et
c) assujettissement de l'extrémité distale de la lame PZT (22) à la pièce monolithique entre les deux surfaces longitudinales opposées (74) de la fente axiale (64) dans la zone de serrage (76 ; 80).

11. Le procédé de la revendication 10, dans lequel à l'étape a) la formation de la fente axiale (64) dans la pièce monolithique est effectuée par une technique d'enlèvement de matière parmi électroérosion à fil ou usinage avec un disque ; ou par une technique additive de dépôt de matière parmi stéréolithographie, SLA, frittage sélectif par laser, SLS, ou dépôt de fil fondu, FDM.

12. Le procédé de la revendication 10, dans lequel à l'étape c) l'assujettissement de l'extrémité distale de la lame PZT (22) à la pièce monolithique est effectué par une technique parmi : déformation en température de la lame PZT (22) ou de la pièce monolithique avant l'insertion de l'étape b) et retour à température ambiante après l'insertion de l'étape b) ; déformation élastique de la pièce monolithique pour agrandir la fente axiale (64) avant l'insertion de l'étape b) et relâchement après l'insertion de l'étape b) ; collage ; sertissage ; et/ou soudure d'une pièce rapportée de liaison.

13. Un module de récupération d'énergie, PEH, comprenant :
- un tube enveloppe (50) allongé ;
- logé à l'intérieur du tube (50), un ensemble pendulaire (22, 26) selon l'une des revendications 1 à 9.

14. Un dispositif autonome logeant, dans un corps de dispositif :
- un ensemble électronique (28-38) ;
- un module PEH (40) selon la revendication 13, produisant en sortie un signal électrique ;
- un circuit de gestion d'alimentation (42), apte à redresser et réguler le signal électrique produit par le module PEH pour délivrer en sortie une tension ou un courant continu d'alimentation stabilisés ; et
- un organe de stockage d'énergie (44) pour l'alimentation de l'ensemble électronique,
dans lequel ladite tension ou ledit courant continu stabilisés délivrés par le circuit de gestion d'alimentation servent à l'alimentation de l'ensemble électronique et/ou à la recharge de l'organe de stockage d'énergie du dispositif autonome.

15. Le dispositif autonome de la revendication 14,
dans lequel le dispositif autonome est un dispositif médical actif de type capsule autonome implantable (10) comprenant un corps de capsule (12) avec un élément d'ancrage (16) à une paroi d'un organe d'un patient,
et dans lequel les sollicitations externes auxquelles est soumis l'ensemble pendulaire (22, 26) du module PEH sont des sollicitations appliquées au corps de capsule (12) sous l'effet de mouvements de ladite paroi et/ou de variations de débit d'un flux dans le milieu environnant.

## Patentansprüche

1. Eine Pendelanordnung für ein Energierückgewinnungsmodul, PEH, wobei die Pendelanordnung umfasst:
- ein Längsorgan (22) eines piezoelektrischen Wandlers oder Blei-Zirkonat-Titanat-Wandlers, PZT, (22), das durch Biegung elastisch verformbar ist und sich in axialer Richtung zwischen einem verankerten proximalen Ende und einem freien distalen Ende erstreckt; und
- eine inertiale Masse (26), die an dem freien distalen Ende des PZT-Längsorgans (22) gelagert und in Querrichtung beweglich ist,
wobei die Pendelanordnung imstande ist, eine mechanische Energie, die von Schwingungen der Pendelanordnung unter der Einwirkung von äußeren Belastungen, die das Modul erfährt, erzeugt wird, in ein oszillierendes elektrisches Signal umzuwandeln, das von Oberflächenelektroden des PZT-Längsorgans (22) empfangen wird, wobei die inertiale Masse (26) ein monolithisches Teil ist, das eine Vertiefung in Form eines axialen Schlitzes (64) aufweist, mit zwei gegenüberliegenden Längsflächen (74), die sich entlang einer mittigen Achse der inertialen Masse (26) erstrecken, wobei der axiale Schlitz (64) auf der proximalen Seite der inertialen Masse (26) ausmündet und das freie distale Ende des PZT-Längsorgans (22) aufnimmt,
**dadurch gekennzeichnet, dass** die Vertiefung in Form eines axialen Schlitzes (64) umfasst:
- auf der proximalen Seite (66) eine Klemmzone (76; 80), wo das PZT-Längsorgan (22) zwischen den zwei gegenüberliegenden Längsflächen (74) des axialen Schlitzes befestigt ist; und
- auf der distalen Seite (68) eine klemmungsfreie Zone (78; 82).

2. Die Pendelanordnung nach Anspruch 1, wobei über die Länge der Klemmzone (76) die zwei gegenüberliegenden Längsflächen (74) ebene und parallele symmetrische Flächen umfassen, die durch einen konstanten radialen Abstand getrennt sind.

3. Die Pendelanordnung nach Anspruch 2, wobei die zwei gegenüberliegenden Längsflächen (74) Flächen umfassen, die sich am Ort der Länge der klemmungsfreien Zone (78) verbreitern.

4. Die Pendelanordnung nach Anspruch 1, wobei die zwei gegenüberliegenden Längsflächen (74) symmetrische Flächen sind, die durch einen radialen Abstand getrennt sind, der in der proximalen zur distalen Richtung über mindestens einen Teil der Länge des axialen Schlitzes (64) in Längsrichtung zunimmt, so dass eine progressive Klemmung des PZT-Längsorgans (22) bewirkt wird, mit einer maximalen Klemmkraft in der Klemmzone (80) und einer Klemmkraft gleich null in der klemmungsfreien Zone (82).

5. Pendelanordnung nach Anspruch 1, wobei in radialer Richtung der axiale Schlitz (64) auch auf mindestens einer der Seiten der inertialen Masse (26) ausmündet.

6. Die Pendelanordnung nach Anspruch 1, wobei mindestens eine der zwei gegenüberliegenden Längsflächen (74) mindestens eine Anti-Kickback-Kerbe (86, 90) umfasst, die mit einem axialen Anschlag (86b) und/oder radialen Anschlag (90b) versehen ist, die zur Lagesicherung des PZT-Längsorgans (22) in dem axialen Schlitz (64) imstande ist.

7. Die Pendelanordnung nach Anspruch 6, wobei das PZT-Längsorgan (22) in dem Bereich zwischen den gegenüberliegenden Längsflächen (74) des axialen Schlitzes (64) mindestens einen Ausschnitt (88, 92) umfasst, der zum Zusammenwirken mit einem entsprechenden axialen Anschlag (86b) und/oder radialen Anschlag (90b) einer Anti-Kickback-Kerbe (86, 90) der inertialen Masse (26) imstande ist.

8. Pendelanordnung nach Anspruch 1, wobei in der Klemmzone (76) der minimale Wert des radialen Abstands zwischen den gegenüberliegenden Längsflächen (74) auf ein negatives Lagerspiel genau gleich der Dicke des PZT-Längsorgans (22) ist, so dass auf das PZT-Längsorgan (22) eine Klemmkraft zwischen den gegenüberliegenden Flächen ausgeübt wird.

9. Die Pendelanordnung nach Anspruch 8, wobei die Kraft der Klemmung des PZT-Längsorgans (22) durch die gegenüberliegenden Längsflächen (74) zwischen 0,5 und 2 N/mm² beträgt.

10. Ein Verfahren zum Montieren einer Pendelanordnung für ein Energierückgewinnungsmodul, PEH, wobei die Pendelanordnung ein Längsorgan (22) eines piezoelektrischen Wandlers oder Blei-Zirkonat-Titanat-Wandlers, PZT, (22), das durch Biegung elastisch verformbar ist, und eine inertiale Masse (26), die an dem freien distalen Ende des PZT-Längsorgans (22) gelagert und in Querrichtung beweglich ist, umfasst,
aufweisend die folgenden Schritte:
a) Erhalten einer inertialen Masse (26) durch Bildung eines axialen Schlitzes (64) in der Masse eines monolithischen Teils, wobei sich der axiale Schlitz (64) entlang einer mittigen Achse der inertialen Masse (26) von einem proximalen Ende erstreckt, in dem er zwei gegenüberliegende Längsflächen (74) bildet, wobei der axiale Schlitz (64) aufeinander folgende Vertiefungen mit unterschiedlicher Geometrie umfasst, umfassend: auf der proximalen Seite (66) eine Klemmzone (76; 80), wo das PZT-Längsorgan (22) zwischen den zwei gegenüberliegenden Längsflächen (74) des axialen Schlitzes befestigt ist; und auf der distalen Seite (68) eine klemmungsfreie Zone (78; 82);
b) Einsetzen des freien distalen Endes eines PZT-Längsorgans (22) in den axialen Schlitz (64); und
c) Befestigen des freien distalen Endes eines PZT-Längsorgans (22) an dem monolithischen Teil zwischen den zwei gegenüberliegenden Längsflächen (74) des axialen Schlitzes (64) in der Klemmzone (76; 80).

11. Das Verfahren nach Anspruch 10, wobei in Schritt a) die Bildung des axialen Schlitzes (64) in dem monolithischen Teil durch eine Technik des Abtragens von Material aus Drahterodieren oder Bearbeitung mit einer Scheibe; oder durch eine additive Technik des Auftragens von Material aus Stereolithografie, SLA, selektives Lasersintern, SLS, oder Schmelzschichtung, FDM, erfolgt.

12. Das Verfahren nach Anspruch 10, wobei in Schritt c) das Befestigen des freien distalen Endes des PZT-Längsorgans (22) an dem monolithischen Teil durch eine Technik erfolgt aus: Thermoverformen des PZT-Längsorgans (22) oder des monolithischen Teils vor dem Einsetzen in Schritt b) und Rückkehr zur Umgebungstemperatur nach dem Einsetzen in Schritt b); elastisches Verformen des monolithischen Teils, um den axialen Schlitz (64) vor dem Einsetzen in Schritt b) zu vergrößern, und Entspannen nach dem Einsetzen in Schritt b); Kleben; Crimpen; und/oder Schweißen eines Anschlussverbindungsstücks.

13. Ein Modul zur Energierückgewinnung, PEH, umfassend:
- ein langgezogenes Mantelrohr (50);
- im Inneren des Rohrs (50) aufgenommen, eine Pendeleinheit (22, 26) nach einem der Ansprüche 1 bis 9.

14. Eine autonome Vorrichtung, die in einem Vorrichtungskörper Folgendes aufnimmt:
- eine elektronische Anordnung (28-38);
- ein PEH-Modul (40) nach Anspruch 13, das ausgangsseitig ein elektrisches Signal erzeugt;
- eine Versorgungsverwaltungsschaltung (42), die dafür eingerichtet ist, das von dem PEH-Modul erzeugte Signal gleichzurichten und zu regeln, um ausgangsseitig eine stabilisierte Versorgungsgleichspannung oder einen stabilisierten Versorgungsgleichstrom bereitzustellen; und
- ein Energiespeicherorgan (44) für die Versorgung der elektronischen Anordnung,
wobei die stabilisierte Gleichspannung oder der stabilisierte Gleichstrom, die/der von der Versorgungsverwaltungsschaltung bereitgestellt werden, der Versorgung der elektronischen Anordnung und/oder der Aufladung des Energiespeicherorgans der autonomen Vorrichtung dienen.

15. Die autonome Vorrichtung nach Anspruch 14, wobei die autonome Vorrichtung eine aktive medizinische Vorrichtung des Typs implantierbare autonome Kapsel (10) ist, die einen Kapselkörper (12) mit einem Element (16) zur Verankerung an einer Wand eines Organs eines Patienten umfasst, und wobei die äußeren Belastungen, die die Pendeleinheit (22, 26) des PEH-Moduls erfährt, Belastungen sind, die auf den Kapselkörper (12) unter der Einwirkung von Bewegungen der Wand und/oder von Veränderungen einer Blutflussrate im umgebenden Medium aufgebracht werden.

## Claims

1. A pendular unit for an energy harvesting module, PEH, the pendular unit comprising:
- a piezoelectric or lead zirconate titanate, PZT, transducer beam (22) that is elastically deformable in bending and that extends in axial direction between a clamped proximal end and a free distal end; and
- an inertial mass (26), mounted at the free distal end of the PZT beam (22) and mobile in transverse direction,
the pendular unit being adapted to convert a mechanical energy produced by oscillations of the pendular unit under the effect of external stresses undergone by the module into an oscillating electrical signal collected by surface electrodes of the PZT beam (22),
wherein the inertial mass (26) is a monolithic part including a cavity in the form of an axial slit (64), with two opposite longitudinal surfaces (74) extending along a central axis of the inertial mass (26), the axial slit (64) opening out on the proximal side of the inertial mass (26) and receiving the free distal end of the PZT beam (22),
**characterized in that** said cavity in the form of an axial slit (64) comprises:
- on a proximal side (66), a clamping area (76; 80) in which the PZT beam (22) is secured between the two opposite longitudinal surfaces (74) of the axial slit; and
- on a distal side (68), a non-clamping area (78; 82).

2. The pendular unit of claim 1, wherein, over the length of the clamping area (76), the two opposite longitudinal surfaces (74) comprise flat and parallel symmetrical surfaces separated by a constant radial spacing.

3. The pendular unit of claim 2, wherein the two opposite longitudinal surfaces (74) comprise surfaces that widen along the length of the non-clamping area (78).

4. The pendular unit of claim 1, wherein the two opposite longitudinal surfaces (74) are symmetrical surfaces radially separated by an increasing radial spacing, in the direction proximal to distal, along at least part of the length of the axial slit (64) in the longitudinal direction, in such a way as to produce a progressive clamping of the PZT beam (22), with a maximum clamping force in the clamping area (80) and zero clamping force in the non-clamping area (82).

5. The pendular unit of claim 1, wherein, in a radial direction, the axial slit (64) also opens out in at least one of a side of the inertial mass (26).

6. The pendular unit of claim 1, wherein at least one of the two opposite longitudinal surfaces (74) comprise at least one non-return notch (86, 90) provided with an axial (86b) and/or radial (90b) stop adapted to block the PZT beam (22) in position in the axial slit (64).

7. The pendular unit of claim 6, wherein the PZT beam (22) comprises, in an area located between the opposite longitudinal surfaces (74) of the axial slit (64), at least one cut (88, 92) adapted to cooperate with an axial (86b) and/or radial (90b) stop for mating a non-return notch (86, 90) of the inertial mass (26).

8. The pendular unit of claim 1, wherein, in the clamping area (76), a minimum value of the radial spacing between the opposite longitudinal surfaces (74) is equal to the thickness of the PZT beam (22), to within a negative clearance, in such a way as to exert on the PZT beam (22) a pinching force between the opposite surfaces.

9. The pendular unit of claim 8, wherein a pinching force of the PZT beam (22) exerted by the opposite longitudinal surfaces (74) is between 0.5 and 2 N/mm².

10. A method for assembling a pendular unit for an energy harvesting module, PEH, the pendular unit comprising a piezoelectric or lead zirconate titanate, PZT, transducer beam (22) that is elastically deformable in bending and an inertial mass 26) that is mounted at a free distal end of the PZT beam (22) and mobile in a transverse direction,
comprising the following steps:
a) obtaining an inertial mass (26) by forming an axial slit (64) in a mass of a monolithic part, the axial slit (64) extending along a central axis of the inertial mass (26) from a proximal end, forming two opposite longitudinal surfaces (74),
the axial slit (64) comprising successive cavities with different geometries, comprising: on a proximal side (66), a clamping area (76; 80) in which the PZT beam (22) is secured between the two opposite longitudinal surfaces (74) of the axial slit; and on a distal side (68), a non-clamping area (78; 82);
b) inserting into the axial slit (64) the free distal end of a PZT beam (22); and
c) securing the distal end of the PZT beam (22) to the monolithic part between the two opposite longitudinal surfaces (74) of the axial slit (64) in the non-clamping area (76; 80).

11. The method of claim 10, wherein, in step a), the formation of the axial slit (64) in the monolithic part is performed using a material removal technique selected among wire electro-erosion or disk machining; or using an additive material deposition technique among stereolithography, SLA, selective laser sintering, SLS, or fused deposition modeling, FDM.

12. The method of claim 10, wherein, in step c), the securing of the distal end of the PZT beam (22) to the monolithic part is performed using a technique among: temperature deformation of the PZT beam (22) or of the monolithic part before insertion in step b) then return to room temperature after insertion in step b); elastic deformation of the monolithic part to enlarge the axial slit (64) before insertion in step b) then release after the insertion in step b); bonding; crimping; and/or welding of an added attachment part.

13. An energy harvesting module, PEH, comprising:
- an elongated envelope tube (50);
- contained in the tube (50), a pendular unit (22, 26) according to any one of claims 1 to 9.

14. An autonomous device containing, in a device body:
- an electronic unit (28-38);
- a PEH module (40) according to claim 13, providing an electric signal;
- a power management circuit (42), adapted to rectify and regulate the electric signal produced by the PEH module, to output a stabilized direct power voltage or current; and
- an energy storage component (44) for powering the electronic unit,
wherein said stabilized direct voltage or current provided by the power management circuit is used to power the electronic unit and/or to charge the energy storage component of the autonomous device.

15. The autonomous device of claim 14,
wherein the autonomous device is an active medical device of the implantable autonomous capsule type (10) comprising a capsule body (12) with an element (16) for anchoring to a wall of a patient's organ,
and wherein the external stresses to which is subjected the pendular unit (22, 26) of the PEH module are stresses applied to the capsule body (12) under the effect of movements of said wall and/or flow rate variations of a flow in the surrounding environment.
